(19) Europäisches Patentamt
European Patent Office
Office européen des brevets

(11) Veröffentlichungsnummer : **0 445 608 B1**

(12) **EUROPÄISCHE PATENTSCHRIFT**

(45) Veröffentlichungstag der Patentschrift : **08.06.94 Patentblatt 94/23**

(51) Int. Cl.$^5$ : **C07C 265/14,** C07C 263/18, C08K 5/09

(21) Anmeldenummer : **91102638.3**

(22) Anmeldetag : **22.02.91**

# (54) Stabilisierung von organischen Polyisocyanaten.

(30) Priorität : **07.03.90 DE 4007074**
**22.12.90 DE 4041516**

(43) Veröffentlichungstag der Anmeldung : **11.09.91 Patentblatt 91/37**

(45) Bekanntmachung des Hinweises auf die Patenterteilung : **08.06.94 Patentblatt 94/23**

(84) Benannte Vertragsstaaten : **BE DE ES FR GB IT NL**

(56) Entgegenhaltungen :
**DE-A- 1 618 845**
**DE-A- 3 816 118**
**FR-A- 2 126 382**
**US-A- 3 281 444**

(73) Patentinhaber : **BAYER AG**
**D-51368 Leverkusen (DE)**

(72) Erfinder : **Gupta, Pramod, Dr.**
**Langemarck Strasse 27**
**W-5012 Bedburg (DE)**
Erfinder : **König, Christian, Dr.**
**Pillauer Weg 11**
**W-4044 Kaarst 1 (DE)**
Erfinder : **Rabe, Hans-Jürgen, Dr.**
**Domblick 20**
**W-5090 Leverkusen 3 (DE)**
Erfinder : **Engels, Hans-Wilhelm, Dr.**
**Dickeicher Feld 12**
**W-5014 Kerpen 3 (DE)**
Erfinder : **Nolte, Wilfried, Dr.**
**Im Altendriesch 18**
**W-5068 Odenthal (DE)**
Erfinder : **Podder, Chiraranjan, Dr.**
**Pommernallee 3**
**W-4047 Dormagen 1 (DE)**

**EP 0 445 608 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft die Verwendung von am Kern substituierten 4-Hydroxyphenyl-propionsäure-Verbindungen zur Stabilisierung von organischen Polyisocyanaten.

Die organischen Isocyanate haben für die Herstellung von Polyurethan-Kunststoffen hohe Bedeutung erlangt. So werden z.B. die organischen Polyisocyanate mit Polyolen (Polyether und Polyester) für Schaumstoff-, Faser-, Film-, Elastomeren- und Lack-Herstellung eingesetzt.

Die organischen Polyisocyanate neigen jedoch bei Lagerung, auch bei niedrigen Temperaturen, zur Verfärbung. Diese Eigenschaft ist besonders ausgeprägt, wenn die Lagerung bei höheren Temperaturen erfolgen muß, z.B. wenn die festen Polyisocyanate für eine homogene Reaktionsführung mit Reaktionspartnern wie z.B. Polyetherpolyolen, Polyesterpolyolen oder Glykolen zu Polyurethanen umgesetzt werden sollen. Auch bei der Polymerherstellung muß die NCO-OH-Reaktion bei höheren Temperaturen durchgeführt werden. Dabei wird beobachtet, daß die Isocyanate sich sehr schnell verfärben, wenn keine Stabilisierung vorgenommen wurde.

Es wurde bereits vorgeschlagen, verschiedene Stabilisatoren organischen Isocyanaten zuzusetzen, um die Verfärbungsneigung der organischen Polyisocyanate zu verringern. Bekannte Stabilisatoren sind sterisch gehinderte Phenole, Dialkyldiphenylamine, Phenothiazine, Phosphite bzw. Gemische von Vertretern aus diesen Substanzklassen (vergl. z.B. US-PS 3 715 301, US-PS 4 064 157, DT-OS 1 668 275, DT-AS 1 618 845).

Am häufigsten findet 2,6-Di-tert.-butyl-4-methylphenol (BHT) allein oder in Kombination mit weiteren Verbindungen der angeführten Stabilisatorenklassen Verwendung zur Stabilisierung von organischen Polyisocyanaten.

Nachteil von BHT ist die relativ hohe Flüchtigkeit und Migrationstendenz in Polyurethane umhüllende Substrate sowie die daraus resultierende starke Gelbfärbung der Substrate in NOx-belasteter Atmosphäre. Stabilisatoren ohne diese Nachteile sind daher von Interesse, und es war Aufgabe der vorliegenden Erfindung, solche Materialien bereitzustellen.

Metallsalze von 3,5-Di-tert.-butyl-4-hydroxyphenylpropionsäure werden in DE-OS 2 209 102 zur Stabilisierung von organischem Material beschrieben, wobei die substratabhängige Wirksamkeit entscheident vom verwendeten Metallatom abhängt.

Gegenstand der vorliegenden Erfindung ist die Verwendung von Verbindungen der allgemeinen Formel:

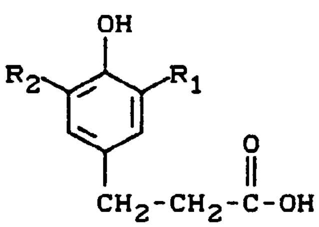

wobei

$R_1$ und $R_2$ für $C_1$- bis $C_8$-Alkylreste, vorzugsweise für $C_1$- bis $C_4$-Alkylreste, stehen und $R_1$ und $R_2$ gleich oder unterschiedlich sind, als Stabilisatoren für organische Polyisocyanate, insbesondere aromatische Polyisocyanate.

Obwohl freie Carboxylgruppen als Substituenten normalerweise nicht zu Stabilisatoren besonderer Wirksamkeit führen und deshalb in aller Regel Derivate von Carbonsäuren, wie Ester, Amide, Hydrazide, etc., verwendet werden, wurde jetzt überraschenderweise gefunden, daß sich die Verbindungen der Klasse am Kern substituierten 4-Hydroxyphenylpropionsäuren wie z.B. 3,5-Di-tert.-butyl-4-hydroxyphenylpropionsäure ausgezeichnet als (stabilisierendes) Antioxidans für die organischen Polyisocyanate eignet.

Je nach Grundaufbau des Polyisocyanates sind auch Kombinationen mit herkömmlichen Antioxidantien wirksam, wobei im allgemeinen die Verbindungen dieser Klasse (wie z.B. 3,5-Di-tert.-butyl-4-hydroxy-phenylpropionsäure) in Mengen von 0,003 bis 1,0 Gew.-%, vorzugsweise 0,003 bis 0,5 Gew.-%, und bei Kombinationsstabilisatoren von 0,003 bis 0,5 Gew.-%, bezogen auf das Polyisocyanat zum Einsatz gelangen können.

Die am Kern substituierten 4-Hydroxyphenylpropionsäure-Verbindungen, die durch basisch katalysierte Addition von Acrylsäuremethylester an substituierte Phenole und anschließende Verseifung (siehe DE 2 120 285) hergestellt werden, eignen sich bei allen gebräuchlichen Polyisocyanaten, einschließlich aliphatischen, aromatischen und cycloaliphatischen Polyisocyanaten. Als Beispiel sind zu erwähnen: Ethylendiisocyanat, Tetramethylendiisocyanat, Hexamethylendiisocyanat, Cyclohexyldiisocyanat, 4,4'-Methylen-bis-(cyclohexylisocyanat), m-Phenylendiisocyanat, p-Phenylendiisocyanat, Toluylen-2,4-diisocyanat, Toluylen-2,6-diisocyanat,

4,4'-Methylen-bis-(phenylisocyanat), 2,2'-Methylen-bis-(phenylisocyanat), 2,3-Methylen-bis-(phenylisocyanat), Toluylen-2,4,6-triisocyanat.

Die so stabilisierten organischen Polyisocyanate zeigen eine sehr stark verminderte Neigung zur Verfärbung bei Lagerung bei erhöhten Temperaturen und können mit Vorteil für die Herstellung von Polyurethanen eingesetzt werden. Die Polyurethane werden ihrerseits für die Schaum-, Film-, Lack- oder Elastomerherstellung verwendet.

Der Gegenstand der vorliegenden Erfindung soll anhand der folgenden Beispiele noch näher erläutert werden (%-Angaben bedeuten jeweils Gew.-% sowie alle Temperaturangaben °C). Die Farbzahl (FZ) APHA wurde jeweils nach DIN 53409 (Juli '67) bzw. ISO (Juli '88) bestimmt.

Beispiele

Beispiel 1

Eine Mischung aus 80 % Toluylen-2,4-diisocyanat und 20 % Toluylen-2,6-diisocyanat wurde mit den nachfolgend angegebenen Mengen an Zusätzen vermischt.

| FZ (APHA) nach | 6 | 15 | 21 Tagen |
|---|---|---|---|
| Desmodur® T 80 (Toluoldiisocyanat; 2,4 80 %; 2,6 20 %) | | | |
| ohne Zusatz | 10 | 50 | 1000 |
| mit Zusatz von 30 ppm BHT 1) | - | - | 250 |
| " " " 100 ppm BHT | - | - | 150 |
| " " " 30 ppm BHP 2) | - | - | 150 |
| " " " 100 ppm BHP | - | - | 150 |

Aus dieser Zusammenstellung geht hervor, daß 30 ppm BHP-Zusatz eine ähnliche Stabilisatorwirkung zeigt wie 100 ppm Zusatz von BHT.

1)

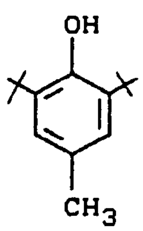

3,5-Di-tert.-Butyl-4-Hydroxy-Toluol

2)

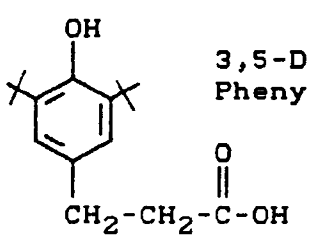

3,5-Di-tert.-Butyl-4-Hydroxy-Phenylpropionsäure

Beispiel 2

Diphenylmethylmethandiisocyanat (Desmodur® 44) (DPMMD) wurde in folgenden Mengen mit dem erfindungsgemäßen Stabilisator vermischt:

Probe A: 500 g DPMMD + 50 mg BHT 1)

Probe B: 500 g DPMMD + 50 mg BHP 2)

Probe C: DPMMD ohne Zusatz

Die Proben wurden einem UV-Bestrahlungstest unterzogen, um das Vergilbungsverhalten zu untersuchen. Nach 70 Stunden Belichtung wurden von diesen Proben Farbzahlen nach APHA gemessen.

| | FZ / APHA |
|---|---|
| Probe A: | 100 |
| Probe B: | 60 |
| Probe C: | 250 |
| vor der Bestrahlung: | 0 bis 5 |

1)

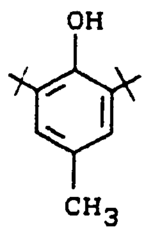

3,5-Di-tert.-Butyl-4-Hydroxy-Toluol

2)

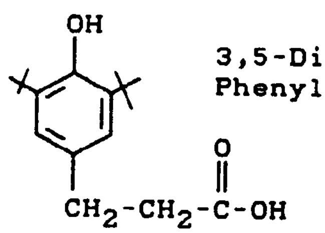

3,5-Di-tert.-Butyl-4-Hydroxy-Phenylpropionsäure

Beispiel 3

Ein Isocyanat wie in Beispiel 1 wurde mit folgenden Zusätzen vermischt:

| | FZ (APHA) nach 3 | 7 | 12 | 18 Tagen |
|---|---|---|---|---|
| mit Zusatz von 30 ppm BHT 1) | - | - | 5 | 150 |
| " " " 30 ppm DHP 2) | - | - | 5 | 150 |

1)

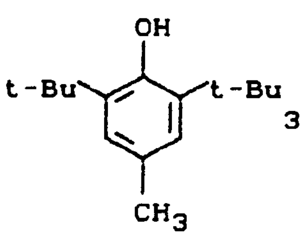

3,5-Di-tert.-Butyl-4-Hydroxy-Toluol

2) 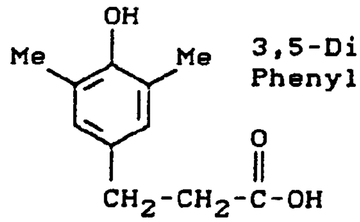 3,5-Di-Methyl-4-Hydroxy-Phenylpropionsäure

## Patentansprüche

1. Verwendung der Verbindungen der allgemeinen Formel

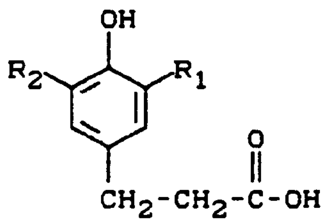

wobei

$R_1$ und $R_2$ für Alkylreste mit 1 bis 8 C-Atomen stehen und $R_1$ und $R_2$ gleich oder unterschiedlich sind, als Stabilisatoren für organische Polyisocyanate, insbesondere aromatische Polyisocyanate.

2. Verfahren zur Herstellung von Polyurethanen, dadurch gekennzeichnet, daß organische Polyisocyanate eingesetzt werden, die durch Zusatz von Verbindungen der allgemeinen Formel gemäß Verwendung nach Anspruch 1 eingesetzt werden.

## Claims

1. The use of compounds corresponding to the following general formula:

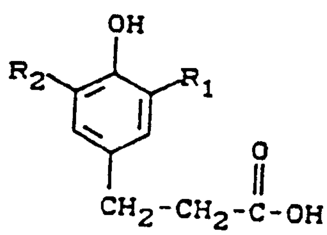

in which

$R_1$ and $R_2$ may be the same or different and represent $C_{1-8}$ alkyl radicals,

as stabilizers for organic polyisocyanates, more particularly aromatic polyisocyanates.

2. A process for the production of polyurethanes, characterized in that organic polyisocyanates stabilized by the addition of compounds corresponding to the general formula in claim 1 are used.

## Revendications

1. Utilisation de composés de formule générale:

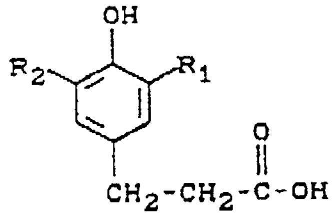

dans laquelle

$R_1$ et $R_2$ représentent des radicaux alkyle en $C_1$-$C_8$ et $R_1$ et $R_2$ peuvent être identiques ou différents, en tant que stabilisants pour des polyisocyanates organiques, en particulier des polyisocyanates aromatiques.

2. Procédé de préparation de polyuréthannes caractérisé en ce qu'on utilise des polyisocyanates organiques qui sont utilisés grâce à l'addition de composés de formule générale selon l'utilisation de la revendication 1.